# EUROPEAN PATENT APPLICATION

(11) **EP 3 281 602 A1**
(43) Date of publication of application: **14.02.2018**
(21) Application number: 16776555.1
(22) Date of filing: 06.04.2016
(51) Int. Cl.: A61B 90/00, A61B 5/00, A61C 19/00, A61C 19/04

(54) **MEDICAL DIAGNOSTIC APPARATUS**

(30) Priority: 07.04.2015 JP 2015078312
(71) Applicant: J. Morita Manufacturing Corporation, Kyoto-shi, Kyoto 612-8533 (JP)
(72) Inventor: SONOBE, Kouichi, Kyoto-shi, Kyoto 612-8533 (JP)
(74) Representative: Müller Hoffmann & Partner
(86) International application number: PCT/JP2016/061243
(87) International publication number: WO 2016/163389

(57) **Abstract**

In a medical care apparatus (10), a host substrate (13) obtains comprehensive update data including pieces of update data of all attachable substrates, specifies a client substrate (11, 12, 14, 15, 16, 17) attached to the medical care apparatus (10), specifies the update data corresponding to the client substrate from the comprehensive update data, determines whether or not execution of update processing is necessary based on a program and the like of the client substrate and the update data, and sends at least the specified update data to the client substrate when determining that execution of the update processing is necessary. The client substrate executes the update processing based on the sent update data.

## Description

### TECHNICAL FIELD

The present invention relates to a medical care apparatus, and particularly to a medical care apparatus in which a plurality of functions are selected and combined.

### BACKGROUND ART

Regarding care in the medical field such as dentistry and medical departments, there has been conventionally known a medical care apparatus in which a plurality of functions can be selected and combined.

For example, a medical care apparatus disclosed in Japanese Patent Laying-Open No. 2002-336281 (PTD 1) can be used for dental care, and function modules having, as functions for the dental care, a root canal length measuring function of measuring a length of a root canal and a root canal enlarging function of enlarging the root canal can be attached to the medical care apparatus. There can be provided a medical care apparatus in which a plurality of functions are selected and combined as described above, and thus, various functions adapted to the intention of a doctor who is a main user, the contents of medical care, the usage environment and the like are performed.

### CITATION LIST

### PATENT DOCUMENT

PTD 1: Japanese Patent Laying-Open No. 2002-336281

### SUMMARY OF INVENTION

### TECHNICAL PROBLEM

In order to perform a function in a medical care apparatus, the update processing for updating at least one of a program and a piece of data (hereinafter also referred to as "program and the like") for performing the function based on prescribed update information must be executed in some cases. When the update processing is executed, a function unit for performing the function must be specified and the update information corresponding to the specified function unit must be selected, for example. Unfortunately, however, individual preparation of the update information corresponding to each function unit is complicated, and it takes a long time to execute the update processing individually and sequentially on all of the function units attached to the medical care apparatus. Furthermore, when the function unit is specified incorrectly or the update information is selected incorrectly, the function unit cannot perform the function and the medical care itself may have to be interrupted.

In the medical care apparatus disclosed in PTD 1 as well, it is conceivable that the functions such as the root canal length measuring function and the root canal enlarging function cannot be performed unless the update processing is executed in a function unit such as a substrate provided in the function module. However, the foregoing problem is not taken into consideration.

The present invention has been made to solve the above-described problem and an object of the present invention is to provide a medical care apparatus in which a program and the like can be updated easily and correctly even when a plurality of functions are selected and combined.

### SOLUTION TO PROBLEM

A medical care apparatus according to the present invention is directed to a medical care apparatus in which a plurality of functions are selected and combined, the medical care apparatus including: at least one function unit configured to perform a function; and an update control unit configured to control the at least one function unit to execute update processing for updating at least one of a program and a piece of data that perform the function based on prescribed update information, the update control unit including: an obtaining unit configured to obtain comprehensive update information including the update information of the at least one function unit attachable to the medical care apparatus; a function specifying unit configured to specify the at least one function unit attached to the medical care apparatus; an update information specifying unit configured to specify the update information corresponding to the at least one function unit specified by the function specifying unit from the comprehensive update information obtained by the obtaining unit; and a sending unit configured to send at least the update information specified by the update information specifying unit to the at least one function unit specified by the function specifying unit, the function unit including an updating unit configured to execute the update processing based on the update information sent from the sending unit, the update control unit or the at least one function unit further including an update determining unit configured to determine whether or not execution of the update processing is necessary, based on the at least one of the program and the piece of data as well as the update information specified by the update information specifying unit.

Preferably, the update control unit is provided on a substrate same as a substrate of the at least one function unit attached to the medical care apparatus.

Preferably, the update control unit is provided on a substrate different from a substrate of the at least one function unit attached to the medical care apparatus.

Preferably, the at least one function unit has a plurality of selectable sub-functions, after the sub-function is selected, the at least one function unit performs the sub-function on condition that the update processing has been executed based on the update information corresponding to the selected sub-function, the comprehensive update information includes the update information corresponding to the selectable sub-functions, and the update information specifying unit specifies the update information corresponding to the at least one function unit specified by the function specifying unit and the selected sub-function from the comprehensive update information obtained by the obtaining unit.

Preferably, the at least one function unit has a plurality of specifications that perform the same function, the function specifying unit further specifies the specification of the at least one function unit attached to the medical care apparatus, and the update information specifying unit specifies the update information corresponding to the at least one function unit specified by the function specifying unit and the specification from the comprehensive update information obtained by the obtaining unit.

Preferably, the medical care apparatus further includes a storing unit configured to store specification information that can specify the specification of the at least one function unit, wherein the update information specifying unit specifies the update information corresponding to the at least one function unit specified by the function specifying unit and the specification from the comprehensive update information obtained by the obtaining unit, based on the specification information stored in the storing unit.

Preferably, the at least one function unit includes a basic function unit configured to perform a basic function of the medical care apparatus, and an extended function unit configured to perform an extended function of the medical care apparatus.

Preferably, the at least one function unit includes a first function unit configured to perform a prescribed function, and a second function unit configured to perform a function related to the first function unit, and the second function unit performs the function on condition that the update processing has been executed in the first function unit.

Preferably, the update control unit further includes a validity determining unit configured to determine validity of the update information specified by the update information specifying unit, before the sending unit sends the update information.

Preferably, the update control unit obtains the comprehensive update information by the obtaining unit via a transmission path connected wiredly or wirelessly.

Preferably, the obtaining unit is communicable with an other medical care apparatus and obtains, via communication, the comprehensive update information obtained by the obtaining unit of the other medical care apparatus.

### ADVANTAGEOUS EFFECTS OF INVENTION

According to the medical care apparatus of the present invention, individual preparation of the update information corresponding to each function unit is unnecessary, and individual and sequential execution of the update processing on all of the function units attached to the medical care apparatus is also unnecessary. Furthermore, the function unit is never specified incorrectly and the update information is never selected incorrectly, and thus, the function unit can perform the function normally. As described above, the program and the like can be updated easily and correctly even when a plurality of functions are selected and combined.

### BRIEF DESCRIPTION OF DRAWINGS

Fig. 1 is a schematic diagram showing a configuration of an outer appearance of a medical care apparatus.
Fig. 2 is a block diagram schematically showing a configuration of the interior of the medical care apparatus.
Fig. 3 is a diagram for describing one example of variations of the medical care apparatus corresponding to combinations of attached substrates.
Fig. 4 is a diagram for describing an obtainment path of comprehensive update data for the medical care apparatus.
Fig. 5 is a block diagram schematically showing functions of a host substrate and a client substrate.
Fig. 6 is a conceptual diagram for describing a configuration of the comprehensive update data.
Fig. 7 is a detailed diagram for describing the configuration of the comprehensive update data.
Fig. 8 is a flowchart for describing a main routine of update control executed by the host substrate.
Fig. 9 is a diagram for describing a constituent member table stored in a memory of the host substrate.
Fig. 10 is a flowchart for describing a subroutine of the update control executed by the host substrate.
Fig. 11 is a flowchart for describing a subroutine of the update control executed by the host substrate.
Fig. 12 is a diagram for describing an update example of a program and the like.

### DESCRIPTION OF EMBODIMENTS

An embodiment according to the present invention will be described with reference to the drawings.

In the present embodiment, a medical care apparatus that can be used for dental care will be described as one exemplary form of a medical care apparatus according to the present invention. The medical care apparatus according to the present invention is applicable to medical care not only in dentistry but also in various medical departments such as ophthalmology, otorhinolaryngology, radiology, and veterinary. In addition, medical care includes diagnosis and treatment.

### [Basic Configuration of Medical Care Apparatus 10]

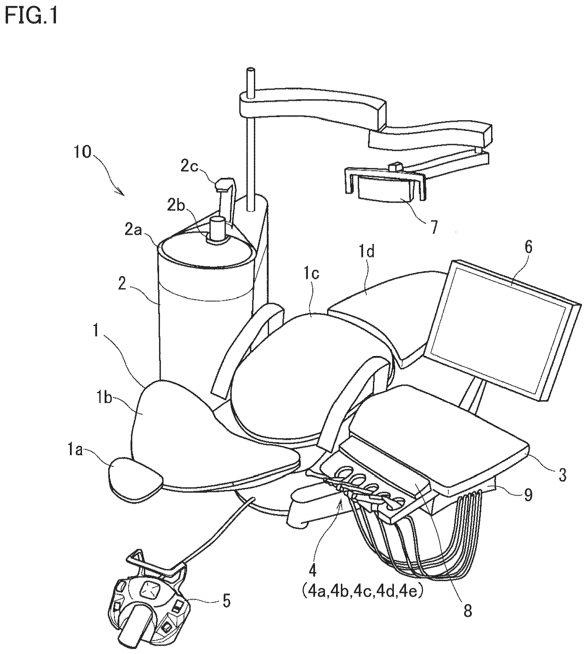

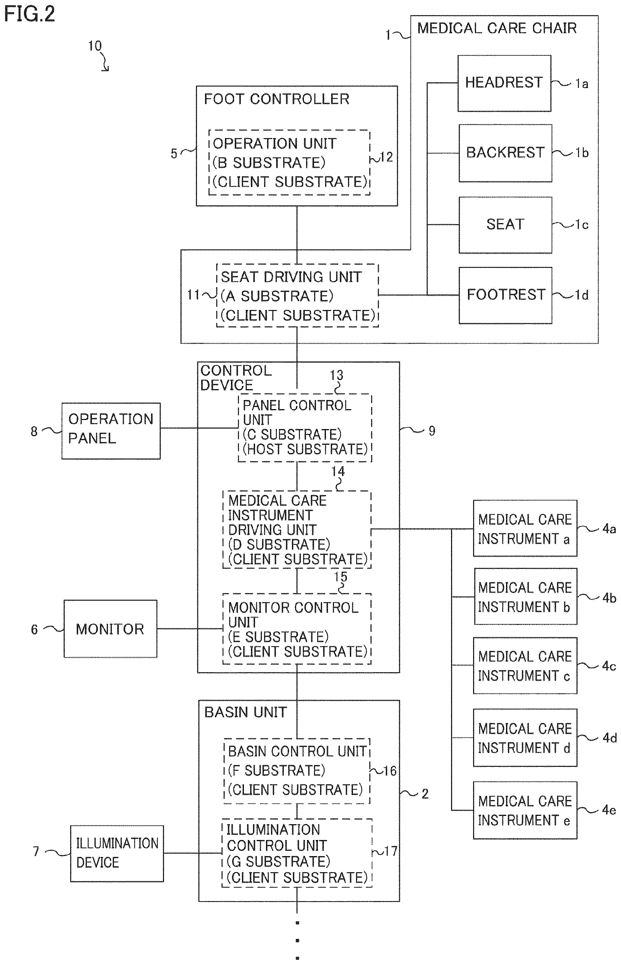

Fig. 1 is a schematic diagram showing a configuration of an outer appearance of a medical care apparatus 10, and Fig. 2 is a block diagram schematically showing a configuration of the interior of medical care apparatus 10. As shown in Figs. 1 and 2, medical care apparatus 10 includes a medical care chair 1, a foot controller 5, a tray table 3, a control device 9, and a basin unit 2.

Medical care chair 1 is a chair on which a patient is seated when receiving medical care from a doctor, and includes a headrest 1a for supporting the head of the patient, a backrest 1b for supporting the back of the patient, a seat 1c for supporting the buttocks of the patient, and a footrest 1d for supporting the legs of the patient. Headrest 1a, backrest 1b, seat 1c, and footrest 1d are connected to a seat driving unit 11 and can be driven based on control by seat driving unit 11. For example, headrest 1a, backrest 1b and footrest 1d can move in a vertical direction or in a horizontal direction with respect to seat 1c based on control by seat driving unit 11. When headrest 1a, backrest 1b and footrest 1d move in the vertical direction, the patient assumes a seated posture. When headrest 1a, backrest 1b and footrest 1d move in the horizontal direction, the patient assumes a face-up posture.

As described above, seat driving unit 11 has the function of driving headrest 1a, backrest 1b and footrest 1d to allow the patient to assume the seated posture or the face-up posture (hereinafter also referred to as "seat driving function"). Seat driving unit 11 corresponds to a computer formed of a CPU (Central Processing Unit), a memory (ROM (Read Only Memory) or RAM (Random Access Memory) (not shown)) and the like mounted on an A substrate. Namely, the seat driving function is performed by the A substrate.

Foot controller 5 is an operation unit for accepting an operation for driving medical care chair 1 and accepting an operation for driving a medical care instrument 4 described below, in accordance with a pressing operation with the foot of the doctor or the like. For example, by pressing a medical care chair driving switch provided on foot controller 5 with the foot, a control signal is output from an operation unit 12 of foot controller 5 to seat driving unit 11, and medical care chair 1 is driven under control by seat driving unit 11. In addition, by pressing a medical care instrument driving switch provided on foot controller 5 with the foot, a control signal is output from operation unit 12 to a medical care instrument driving unit 14, and medical care instrument 4 is driven under control by medical care instrument driving unit 14.

As described above, operation unit 12 has the function of accepting the operations for driving medical care chair 1 and medical care instrument 4 (hereinafter also referred to as "operation accepting function"). Operation unit 12 corresponds to a computer formed of a CPU, a memory (not shown) and the like mounted on a B substrate. Namely, the operation accepting function is performed by the B substrate.

Tray table 3 is used as a stillage during medical care. Tray table 3 is connected to an arm (not shown) extending from medical care chair 1 or a floor, and can be manually pivoted, moved horizontally and moved vertically with respect to medical care chair 1.

Control device 9 is provided on a bottom surface of tray table 3 and has a plurality of medical care instruments 4, an operation panel 8 and a monitor 6 connected thereto. Control device 9 is a control unit for driving these components.

Medical care instrument 4 is an instrument used for medical care, such as, for example, a handpiece like an air turbine handpiece or a micromotor handpiece, or a scaler, a three-way syringe or a vacuum syringe. Medical care instrument 4 is held by an instrument holder (not shown) provided at tray table 3. Medical care instrument 4 is not limited thereto. Medical care instrument 4 may be an intraoral camera, a photopolymerization irradiator, a root canal length measuring device, a root canal enlarging device and the like, or may be a not-driven instrument such as a mirror and a syringe. In the present embodiment, five types of medical care instruments 4a to 4e are used. Each medical care instrument 4 is connected to medical care instrument driving unit 14 of control device 9 and can be driven based on control by medical care instrument driving unit 14. For example, when the handpiece is used as medical care instrument 4, a cutting tool held in a head portion can be rotated and used for medical care based on control by medical care instrument driving unit 14.

As described above, medical care instrument driving unit 14 has the function of driving medical care instrument 4 (hereinafter also referred to as "medical care instrument driving function"). Medical care instrument driving unit 14 corresponds to a computer formed of a CPU, a memory (not shown) and the like mounted on a D substrate. Namely, the medical care instrument driving function is performed by the D substrate.

Operation panel 8 is an operation unit for accepting the operation for driving medical care chair 1 and accepting an operation for making settings related to driving of medical care instrument 4 (e.g., an operation for setting whether to eject water or air, an operation for setting the rotation speed of a motor, and the like), in accordance with the touch operation of a displayed prescribed image (e.g., a character image, an icon image and the like) by the doctor or the like. For example, a medical care chair driving icon image operated to drive medical care chair 1, and a medical care instrument driving icon image operated to make the settings related to driving of medical care instrument 4 are displayed on operation panel 8. When a panel control unit 13 of control device 9 detects the touch operation of the medical care chair driving icon image, panel control unit 13 outputs a control signal to seat driving unit 11. As a result, medical care chair 1 is driven under control by seat driving unit 11. When panel control unit 13 detects the touch operation of the medical care instrument driving icon image, panel control unit 13 outputs a control signal to medical care instrument driving unit 14. As a result, the settings related to driving of medical care instrument 4 are made under control by medical care instrument driving unit 14.

As described above, panel control unit 13 has the function of detecting the touch operation of the prescribed image displayed on operation panel 8 and driving seat driving unit 11 and the function of detecting the touch operation of the prescribed image displayed on operation panel 8 and making the settings related to driving of medical care instrument driving unit 14 (hereinafter also referred to as "panel control function"). Panel control unit 13 corresponds to a computer formed of a CPU, a memory (not shown) and the like mounted on a C substrate. Namely, the panel control function is performed by the C substrate.

Monitor 6 is formed of a liquid crystal display and the like, and displays a prescribed image based on control by a monitor control unit 15 of control device 9. For example, monitor 6 displays an image indicating the driving information of medical care instrument 4 taken out from the instrument holder and used, an image indicating a position of a tip end of the cutting tool in the root canal, an intraoral image taken by the intraoral camera, and the like.

As described above, monitor control unit 15 has the function of controlling the image display on monitor 6 (hereinafter also referred to as "monitor control function"). Monitor control unit 15 corresponds to a computer formed of a CPU, a memory (not shown) and the like mounted on an E substrate. Namely, the monitor control function is performed by the E substrate.

Basin unit 2 is provided on the side portion of medical care chair 1 and includes a basin 2a provided with a water outlet, a cup stand 2b on which a cup is disposed, and a faucet 2c for supplying water to the cup. Basin unit 2 is a stand for the patient to gargle his throat using the water supplied from faucet 2c to the cup.

Basin unit 2 also includes a basin control unit 16 and controls a flow of water used in medical care apparatus 10 based on control by basin control unit 16.

As described above, basin control unit 16 has the function of controlling the flow of water used in medical care apparatus 10 (hereinafter also referred to as "basin control function"). Basin control unit 16 corresponds to a computer formed of a CPU, a memory (not shown) and the like mounted on an F substrate. Namely, the basin control function is performed by the F substrate.

Basin unit 2 further includes an illumination control unit 17 and controls turn-on and off of an illumination device 7 based on control by illumination control unit 17.

As described above, illumination control unit 17 has the function of controlling turn-on and off of illumination device 7 (hereinafter also referred to as "illumination control function"). Illumination control unit 17 corresponds to a computer formed of a CPU, a memory (not shown) and the like mounted on a G substrate. Namely, the illumination control function is performed by the G substrate.

In the example shown in Fig. 2, medical care apparatus 10 includes only the A substrate to the G substrate. However, medical care apparatus 10 may be an apparatus including a substrate other than these substrates and being capable of performing a function other than the above-described functions, or may be an apparatus including not all of the A substrate to the G substrate.

In addition, in medical care apparatus 10, the plurality of substrates that perform the functions include a substrate that performs a basic function essential for dental care, and a substrate that performs an extended function. For example, the A substrate that performs the seat driving function, the B substrate that performs the operation accepting function, the C substrate that performs the panel control function, and the D substrate that performs the medical care instrument driving function are essential for dental care. Therefore, these substrates are normally included as standard equipment from the beginning of delivery after production of medical care apparatus 10. On the other hand, the substrates other than these substrates are substrates that perform the extended functions, and thus, can be added optionally. Therefore, the substrates that perform the extended functions can be attached to or removed from medical care apparatus 10 at the time of delivery or after delivery, depending on the intention of the doctor who is a main user, the contents of medical care, the usage environment and the like.

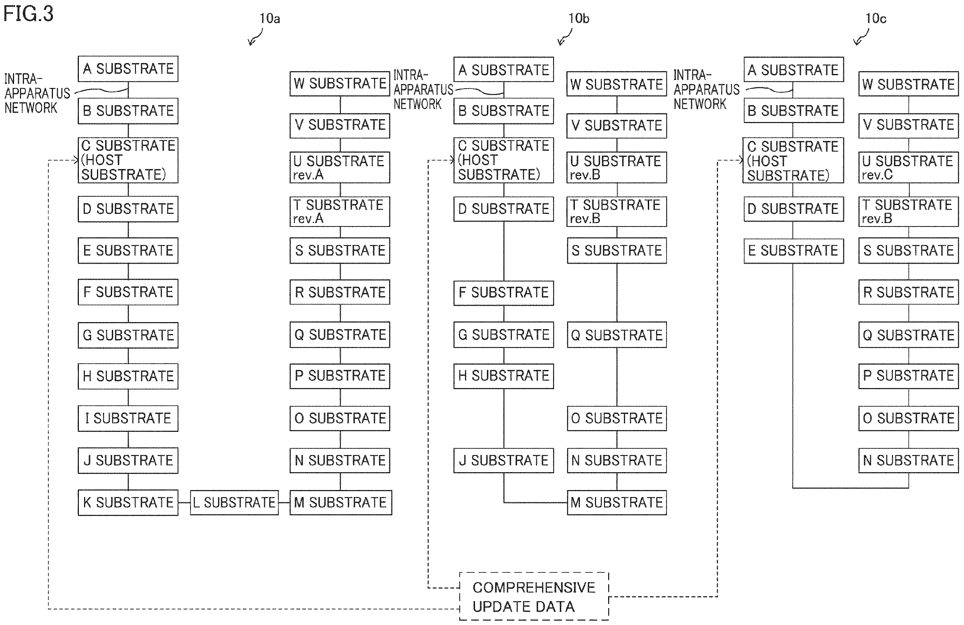

For example, Fig. 3 is a diagram for describing one example of variations of medical care apparatus 10 corresponding to combinations of the attached substrates. In the example of Fig. 3, three types of medical care apparatuses 10a to 10c are shown.

As shown in Fig. 3, all of the attachable substrates, i.e., the A substrate to the W substrate, are attached to medical care apparatus 10a, whereas not all of the substrates are attached to medical care apparatus 10b and medical care apparatus 10c and the substrates are selectively combined. For example, the E substrate is not attached to medical care apparatus 10b, and thus, the monitor control function by the E substrate cannot be performed. The F substrate and the G substrate are not attached to medical care apparatus 10c, and thus, the basin control function by the F substrate and the illumination control function by the G substrate cannot be performed.

As described above, in medical care apparatus 10 of the present embodiment, each function can be performed by each attached substrate, and thus, the doctor can smoothly provide medical care to the patient using medical care apparatus 10. In addition, there can be provided medical care apparatus 10 in which the plurality of functions are selected and combined, and thus, various functions adapted to the intention of the doctor who is a main user, the contents of medical care, the usage environment and the like are performed.

In order for each substrate to perform the function thereof, update processing for updating the program and the like stored in the memory of the substrate based on prescribed update data must be executed in some cases. The program and the like in the present embodiment include at least one of a program and a piece of data. The program refers to a command and processing procedure for causing the computer on the substrate to perform the prescribed operation, i.e., software (also described as "firmware") of the substrate. The piece of data refers to various types of data such as setting data, image data and voice data used when the computer on the substrate performs the prescribed operation. Furthermore, the update data of the present embodiment includes, for example, the latest information about the program and the like. Namely, when the update processing is executed, the program and the like stored in the memory of the substrate are updated (also described as "rewritten") to the latest program and the like in the update data. The case of having to execute the update processing will be described below. The example described below is merely one example, and the update processing may have to be executed for the other reasons in some cases.

As a first example, when some trouble occurs in the program and the like of each substrate, for example, each function cannot be performed unless the program and the like are updated to the normal program and the like. Specifically, when some trouble occurs in the program and the like of the C substrate serving as panel control unit 13, for example, the touch operation of the prescribed image displayed on operation panel 8 cannot be detected in some cases unless the program and the like of the C substrate are updated to the normal program and the like. Even when the touch operation can be detected, the control signal for driving seat driving unit 11 and the control signal for driving medical care instrument driving unit 14 cannot be output in some cases unless the program and the like of the C substrate are updated to the normal program and the like. In addition, when some trouble occurs in the data of the C substrate, an image that is not essentially displayed on monitor 6 may be displayed.

As a second example, at least one substrate has a plurality of selectable sub-functions, and after the sub-function is selected, the substrate can perform the sub-function on condition that the update processing has been executed based on the update data corresponding to the selected sub-function. The medical care instrument driving function performed by the D substrate serving as medical care instrument driving unit 14 includes, for example, a main function of driving the instrument such as a handpiece, a scaler, a three-way syringe, and a vacuum syringe included as standard equipment, and a sub-function of driving the instrument such as an intraoral camera, a photopolymerization irradiator, a root canal length measuring device, and a root canal enlarging device that can be additionally included. A program and the like for performing the main function are stored in the memory of the D substrate from the beginning of delivery of medical care apparatus 10, whereas a program and the like for performing the sub-function are not stored in the memory of the D substrate at the time of delivery of medical care apparatus 10. Therefore, when the instrument such as an intraoral camera, a photopolymerization irradiator, a root canal length measuring device, and a root canal enlarging device is added after delivery of medical care apparatus 10, the medical care instrument driving function cannot be performed on the added instrument unless the program and the like of the D substrate are updated. Not only the program for performing the sub-function but also the program for performing the main function must be changed in some cases, depending on the contents of medical care and the environment. For example, when the specifications of an electric motor are changed, the information for controlling driving also needs to be changed accordingly. In this case as well, the medical care instrument driving function cannot be performed on the changed instrument unless the program and the like of the D substrate are changed.

As a third example, at least one substrate has a plurality of specifications that perform the same function. For example, the C substrate serving as panel control unit 13 has a plurality of specifications such as a specification destined for Japan, a specification destined for Europe and a specification for the Middle East, such that different image displays are obtained on operation panel 8 depending on a difference in language, a difference in uses of the medical care instrument, and the like. Therefore, when the specification of the C substrate is changed, the panel control function corresponding to the specification cannot be performed unless the program and the like of the C substrate are also updated to correspond to the changed specification. In addition, the F substrate serving as basin control unit 16 has a plurality of specifications for a pipeline attached in basin unit 2. Therefore, when the pipeline specification controlled by the F substrate is changed, the flow of water used in medical care apparatus 10 cannot be controlled unless the program and the like of the F substrate are also updated to correspond to the changed pipeline specification.

As a fourth example, at least one substrate performs a function related to another substrate and can perform the function on condition that the update processing has been executed in the other substrate. For example, when medical care instrument 4 is newly added, the C substrate serving as panel control unit 13 cannot recognize the added instrument and perform the panel control function on the added instrument unless the program and the like of the D substrate serving as medical care instrument driving unit 14 are updated.

As a fifth example, the substrate performing the extended function can be attached to or removed from medical care apparatus 10, and thus, it is also conceivable that monitor 6 is not included as standard equipment at the outset of delivery of medical care apparatus 10, while monitor 6 is added after delivery, for example. In this case, the E substrate cannot perform the monitor control function unless not only are monitor 6 and the E substrate serving as monitor control unit 15 attached to medical care apparatus 10 but also the program and the like of the E substrate are updated.

As described above, in order for each substrate to perform the function thereof, the update processing for the program and the like of the substrate must be executed in some cases. When the update processing is executed, the substrate attached to medical care apparatus 10 must be specified and the update data corresponding to the specified substrate must be selected, for example. However, individual preparation of the update data corresponding to each substrate is complicated, and it takes a long time to execute the update processing individually and sequentially on all of the substrates attached to medical care apparatus 10. Furthermore, when the substrate is specified incorrectly or the update data is selected incorrectly, the substrate cannot perform the function and the medical care itself may have to be interrupted.

Thus, in medical care apparatus 10 according to the present embodiment, comprehensive update data including the pieces of update data of all attachable substrates is obtained, the substrate (hereinafter also referred to as "constituent substrate") attached to medical care apparatus 10 is specified, and the update data corresponding to the specified substrate is specified from the obtained comprehensive update data. Furthermore, in medical care apparatus 10, when it is determined that execution of the update processing is necessary, based on the program and the like of the substrate and the specified update data, the update processing is executed based on the specified update data. The update processing executed by medical care apparatus 10 of the present embodiment will be described below. [Configuration Related to Update Processing in Medical Care Apparatus 10]

As shown in Figs. 2 and 3, the constituent substrates of medical care apparatus 10 include a host substrate and a client substrate. The host substrate corresponds to one example of "update control unit", and the client substrate corresponds to one example of "function unit". Each substrate is connected to be communicable with any one of the other substrates via an intra-apparatus network. In the example of Figs. 2 and 3, the host substrate is connected to be communicable only with the adjacent client substrate and is connected to be communicable with the other client substrates through the adjacent client substrate. However, the present invention is not limited to such communication manner. The host substrate may be connected to be directly communicable with all of the other client substrates.

The host substrate is a substrate that executes control for causing each client substrate to execute the update processing for updating the program and the like based on the update data obtained from outside (hereinafter also referred to as "update control"), and the substrate performing the basic function corresponds to the host substrate. In the example shown in Figs. 2 and 3, the C substrate corresponds to the host substrate. As described above, the substrate performing the basic function included as standard equipment is used as the host substrate, and thus, there is no possibility of removal after delivery like the substrate performing the extended function. The client substrate is a substrate that executes the update processing based on the update control by the host substrate, and both of the substrate performing the basic function and the substrate performing the extended function correspond to the client substrate. In the example shown in Figs. 2 and 3, the substrates other than the C substrate correspond to the client substrate.

As shown in Fig. 3, at least one substrate has a plurality of specifications that perform the same function. For example, the T substrate and the U substrate of medical care apparatus 10a have an A specification, and the T substrate and the U substrate of medical care apparatus 10b have a B specification. In addition, the T substrate of medical care apparatus 10c has a C specification, and the U substrate of medical care apparatus 10c has the B specification. The specification includes a hardware specification and a software specification. Specifically, one example of being different in hardware specification is such that a particular component is mounted on the motor circuit in the T substrate of medical care apparatus 10a having the A specification, while a particular component is not mounted on the motor circuit in the T substrate of medical care apparatus 10b having the B specification. One example of being different in software specification is such that the rotation speed of the motor is low and thus software for rotating the motor at high speed is not supported in the T substrate of medical care apparatus 10a having the A specification, while the rotation speed of the motor is high and thus software for rotating the motor at high speed is supported in the T substrate of medical care apparatus 10b having the B specification.

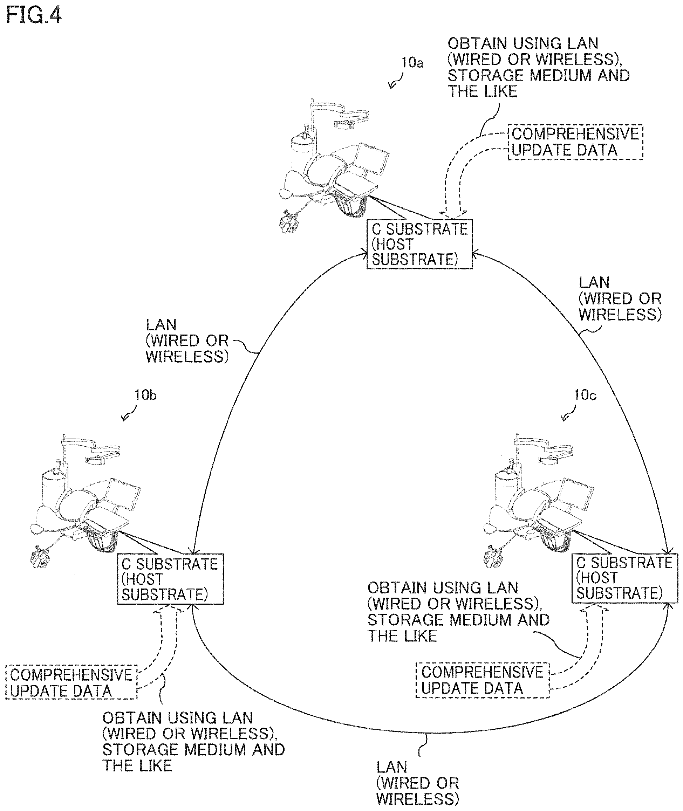

Fig. 4 is a diagram for describing an obtainment path of the comprehensive update data for medical care apparatus 10. As shown in Fig. 4, the C substrate serving as the host substrate can obtain the comprehensive update data from outside via a wired LAN (Local Area Network) or a wireless LAN, or can obtain the comprehensive update data from a storage medium and the like.

Furthermore, the C substrate of medical care apparatus 10 can mutually communicate with the C substrate of another medical care apparatus 10 via the wired LAN or the wireless LAN, and the comprehensive update data obtained by the C substrate of medical care apparatus 10 can also be exchanged with the C substrate of another medical care apparatus 10. For example, the C substrate of medical care apparatus 10a, the C substrate of medical care apparatus 10b and the C substrate of medical care apparatus 10c can communicate with one another via the wired LAN or the wireless LAN, and the comprehensive update data obtained by the C substrate of medical care apparatus 10a from outside can be exchanged with the C substrate of medical care apparatus 10b and the C substrate of medical care apparatus 10c.

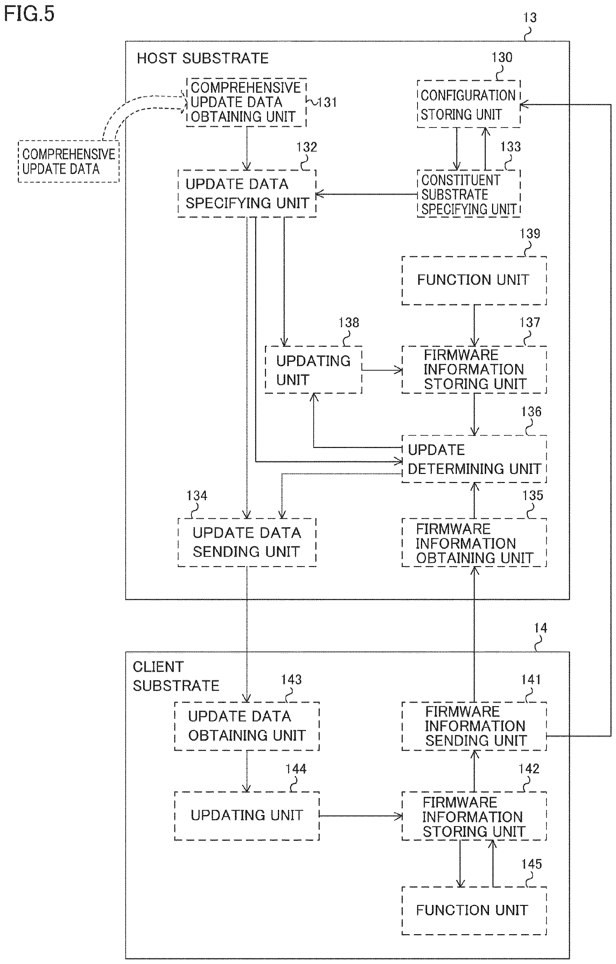

Fig. 5 is a block diagram schematically showing the functions of the host substrate and the client substrate. Fig. 5 shows the example in which the host substrate corresponds to the C substrate serving as panel control unit 13, and the client substrate corresponds to the D substrate serving as medical care instrument driving unit 14. However, even when the other substrates correspond to the host substrate or the client substrate, the substrates have the same functions as the functions shown in Fig. 5. In addition, various functions shown in Fig. 5 are a part of functions, and the host substrate and the client substrate also have the other functions.

The host substrate has a configuration storing unit 130, a comprehensive update data obtaining unit 131, an update data specifying unit 132, a constituent substrate specifying unit 133, an update data sending unit 134, a firmware information obtaining unit 135, an update determining unit 136, a firmware information storing unit 137, an updating unit 138, and a function unit 139.

The client substrate has a firmware information sending unit 141, a firmware information storing unit 142, an update data obtaining unit 143, an updating unit 144, and a function unit 145.

First, the host substrate side will be described. Comprehensive update data obtaining unit 131 corresponds to one example of "obtaining unit" and obtains the comprehensive update data from outside via the wired LAN or the wireless LAN, the storage medium or the like. Comprehensive update data obtaining unit 131 may obtain the comprehensive update data obtained by comprehensive update data obtaining unit 131 of another medical care apparatus 10, via communication by a communicating unit (not shown). Constituent substrate specifying unit 133 corresponds to one example of "function specifying unit" and specifies the client substrate of medical care apparatus 10 based on a constituent member table (details will be described below) stored in configuration storing unit 130. Configuration storing unit 130 corresponds to one example of "storing unit" and has the function of the memory described below.

Update data specifying unit 132 corresponds to one example of "update information specifying unit" and specifies the update data corresponding to the client substrate specified by constituent substrate specifying unit 133 from the comprehensive update data obtained by comprehensive update data obtaining unit 131. Firmware information obtaining unit 135 obtains firmware information sent from firmware information sending unit 141 of the client substrate. The firmware information includes the information that can specify the program and the like of the client substrate.

Update determining unit 136 corresponds to one example of "update determining unit" and determines whether or not execution of the update processing is necessary, based on the firmware information obtained by firmware information obtaining unit 135 and the update data specified by update data specifying unit 132. Specifically, update determining unit 136 determines whether or not the program and the like of the client substrate are the latest, based on the information about the program and the like that can be specified from the firmware information and the information about the program and the like that can be specified from the update data. If the program and the like of the client substrate are not the latest, update determining unit 136 determines that execution of the update processing is necessary. If the program and the like of the client substrate are the latest, update determining unit 136 determines that execution of the update processing is not necessary. Update data sending unit 134 corresponds to one example of "sending unit" and sends the update data specified by update data specifying unit 132 to the client substrate specified by constituent substrate specifying unit 133, when update determining unit 136 determines that execution of the update processing is necessary.

Next, the client substrate side will be described. Update data obtaining unit 143 obtains the update data sent from update data sending unit 134. Updating unit 144 corresponds to one example of "updating unit" and executes the update processing for updating the program and the like stored in firmware information storing unit 142 (corresponding to the function of the memory described below), based on the update data obtained by update data obtaining unit 143. Function unit 145 performs the function based on the program and the like stored in firmware information storing unit 142. For example, in the case of the example of Fig. 5, function unit 145 of medical care instrument driving unit 14 drives medical care instrument 4 based on the program and the like stored in firmware information storing unit 142.

The update processing is also executed in the host substrate. Specifically, update data specifying unit 132 specifies the update data corresponding to the host substrate from the comprehensive update data obtained by comprehensive update data obtaining unit 131. Update determining unit 136 determines whether or not execution of the update processing is necessary, based on the firmware information stored in firmware information storing unit 137 and the update data specified by update data specifying unit 132. When update determining unit 136 determines that execution of the update processing is necessary, updating unit 138 executes the update processing for updating the program and the like stored in firmware information storing unit 137, based on the update data specified by update data specifying unit 132. In addition, function unit 139 performs the function based on the program and the like stored in firmware information storing unit 137. For example, in the case of the example of Fig. 5, function unit 139 of panel control unit 13 detects the touch operation of the prescribed image displayed on operation panel 8, based on the program and the like stored in firmware information storing unit 137, and drives seat driving unit 11 and medical care instrument driving unit 14.

### [Configuration of Comprehensive Update Data]

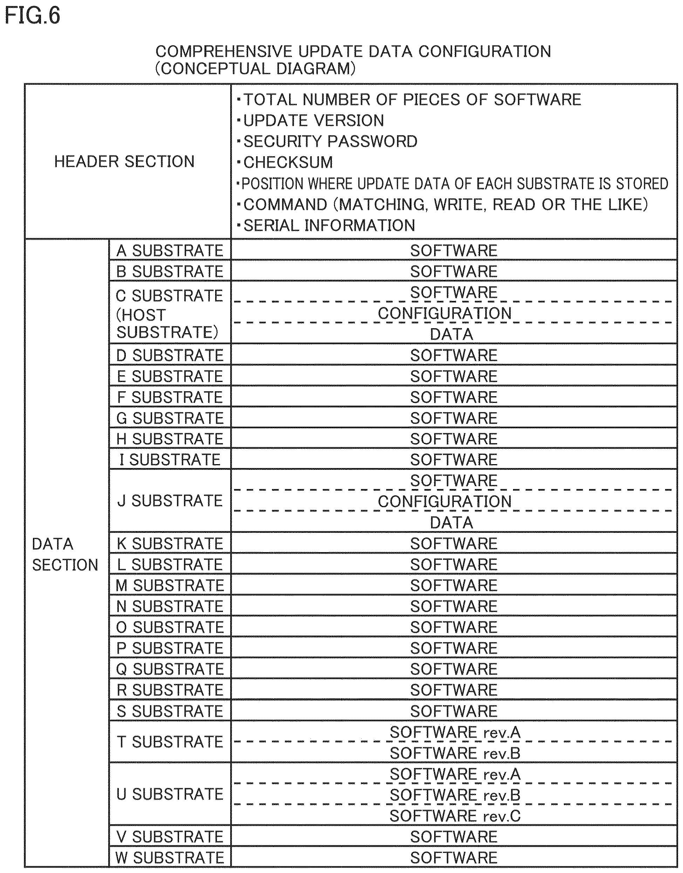

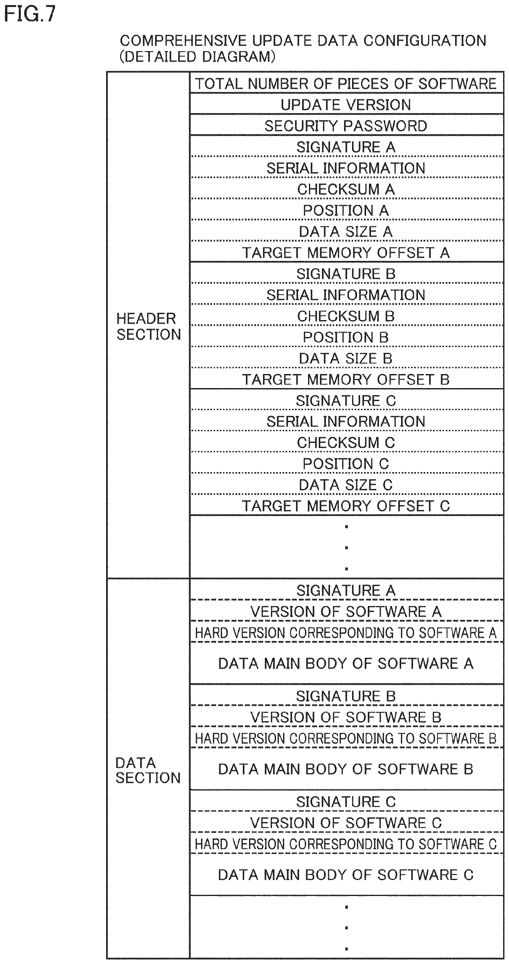

Fig. 6 is a conceptual diagram for describing a configuration of the comprehensive update data. Fig. 7 is a detailed diagram for describing the configuration of the comprehensive update data. As shown in Figs. 6 and 7, the comprehensive update data is formed of a header section and a data section. Furthermore, the header section and the data are stored in the comprehensive update data to correspond to each of all constituent substrates attachable to medical care apparatus 10.

The information about the update data stored in the data section is stored in the header section. For example, the total number of pieces of software stored in the data section, an update version of the comprehensive update data, a security password for permitting reference to the comprehensive update data, a checksum for detecting an error at the time of transmission and reception, a position in the data section where the update data of each substrate is stored, a command for ordering matching, write, read or the like, serial information corresponding to the specification of the update data stored in the data section, and the like are stored in the header section. A serial number (e.g., a production number) of medical care apparatus 10 is, for example, used as the serial information. When the serial information of medical care apparatus 10 is known, the update date corresponding to the specification, of the update data stored in the data section, is known based on this serial information. Namely, the serial information is also described as the information that can specify the specification of the update data stored in the data section.

The latest information about the program and the like is stored in the data section as the update data. For example, pieces of software of all substrates (the A substrate to the W substrate) attachable to medical care apparatus 10, various types of data such as setting data, a configuration which is a program for using a circuit in a programmable device like, for example, FPGA (Field Programmable Gate Array), and the like are stored in the data section.

The signatures in the header section and the data section shown in Fig. 7 are the identification information used when the host substrate specifies the update data of the constituent substrate based on the constituent member table in the memory.

Furthermore, pieces of update data corresponding to all sub-functions selectable in all substrates, like the sub-function selected in the D substrate serving as medical care instrument driving unit 14, are stored in the data section.

As described above, all pieces of update data corresponding to the specifications and the selectable sub-functions of all substrates attachable to medical care apparatus 10 are stored in the comprehensive update data. Therefore, simply by obtaining one piece of comprehensive update data, the pieces of update data corresponding to all substrates, all specifications and all sub-functions can be obtained, and thus, the update processing can be easily executed at a time based on the pieces of update data thus obtained.

### [Flowchart Related to Update Control by Host Substrate]

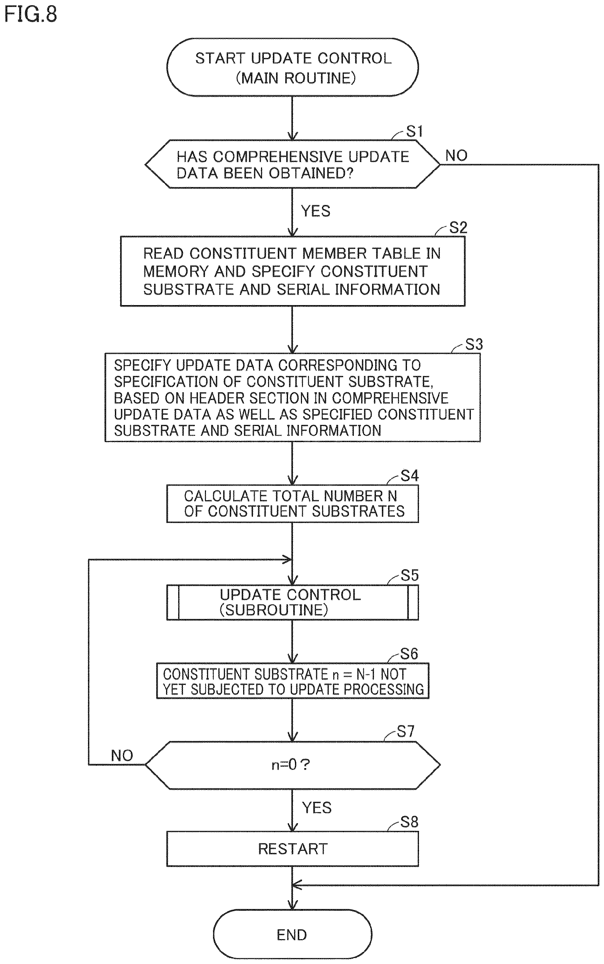

Fig. 8 is a flowchart for describing a main routine of the update control executed by the host substrate. The routine shown in Fig. 8 is a routine when executing the update control for updating the program and the like stored in the memory of the constituent substrate to the latest program and the like. The routine shown in Fig. 8 is authenticated by the security password based on the operator's operation, and then, executed by the host substrate.

As shown in Fig. 8, the host substrate first determines whether or not comprehensive update data obtaining unit 131 has obtained the comprehensive update data from outside (S1). If the host substrate determines that the comprehensive update data has not been obtained (NO in S1), the host substrate ends the routine.

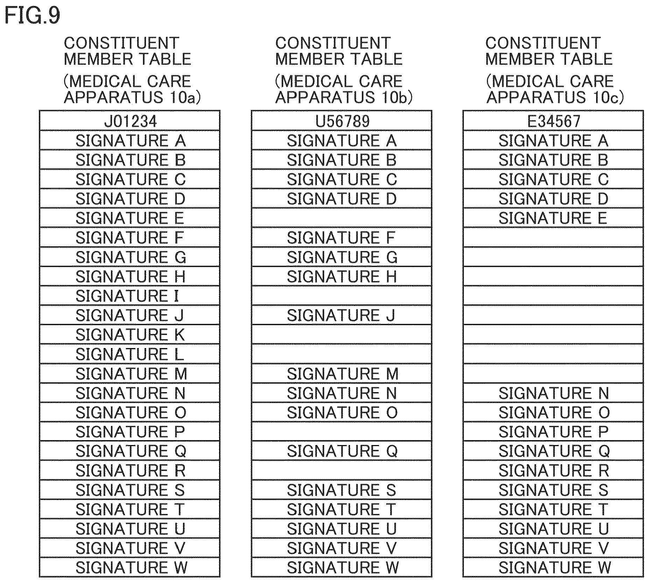

On the other hand, if the comprehensive update data has been obtained (YES in S1), the host substrate reads the constituent member table in the memory, specifies the constituent substrate of medical care apparatus 10 and further specifies the serial information, by constituent substrate specifying unit 133 (S2). Fig. 9 is a diagram for describing the constituent member table stored in the memory of the host substrate. As shown in Fig. 9, the signatures of the constituent substrates attached to medical care apparatus 10 and the serial information are stored in the constituent member table. For example, all of the attachable substrates, i.e., the A substrate to the W substrate, are attached to medical care apparatus 10a, and thus, the signatures corresponding to all of the substrates and the serial information of "J01234" are stored in the constituent member table. The serial information stored in the constituent member table is the information that can specify the specification of the constituent substrate. Therefore, by performing matching between the serial information stored in the constituent member table with the serial information stored in the header section, the update data corresponding to the specification of the constituent substrate can be specified. In other medical care apparatus 10b and medical care apparatus 10c as well, the signatures of the constituent substrates and the serial information are stored in the constituent member table.

The information in the constituent member table is updated by the host substrate when the substrate is attached to or removed from medical care apparatus 10, or when it can be confirmed that the comprehensive update data has a constituent member unknown to the host substrate. This will be described with reference to Fig. 5.

The constituent member table is stored in configuration storing unit 130 having the function of the memory of the host substrate. Namely, the signatures of the constituent substrates and the serial information that can specify the specification of the constituent substrate are stored in configuration storing unit 130 of the host substrate. Configuration storing unit 130 corresponds to one example of "storing unit". The host substrate sends a command for requesting the signature to a portion of connection (not shown) to each client substrate at prescribed intervals (e.g., intervals of 100 msec). The host substrate may send the command for requesting the signature to the portion of connection to each client substrate, when receiving the comprehensive update data. When a client substrate is newly attached to the portion of connection to the host substrate, firmware information sending unit 141 of the client substrate sends a signature to the host substrate in response to the command provided from the host substrate. As a result, the signature of the newly attached client substrate is added to the constituent member table stored in configuration storing unit 130. Namely, the host substrate can regenerate the constituent member table based on the information sent by the client substrate, and thus, the host substrate can hold the latest constituent member table based on the comprehensive update data. On the other hand, when the client substrate attached to the portion of connection to the host substrate is removed, no response is made to the command provided from the host substrate. As a result, the signature of the client substrate removed from the constituent member table stored in configuration storing unit 130 is deleted. As described above, the information in the constituent member table of the host substrate is updated at prescribed intervals (e.g., intervals of 100 msec) or when the comprehensive update data is received.

The serial information is stored in the constituent member table at the time of manufacturing of medical care apparatus 10. However, similarly to the signatures, the serial information may be updated at prescribed intervals (e.g., intervals of 100 msec) when the specification of the attached client substrate is changed.

By referring to the constituent member table in the memory, the host substrate can specify the constituent substrate attached to medical care apparatus 10 and the serial information.

Referring again to Fig. 8, by update data specifying unit 132, the host substrate specifies the update data corresponding to the specification of the constituent substrate in the data section, based on the header section in the comprehensive update data as well as the constituent substrate and the serial information specified from the constituent member table (S3). For example, based on the serial information stored in the constituent member table and the serial information stored in the header section, the host substrate specifies a position where the update data corresponding to the specification of the constituent substrate, of the update data stored in the data section, is stored. Furthermore, the host substrate specifies a position in the data section where the update data of the constituent substrate is stored, based on the signatures of the constituent substrates stored in the constituent member table and the signatures stored in the header section. As a result, the host substrate can specify the update data corresponding to the specification of the constituent substrate from the comprehensive update data.

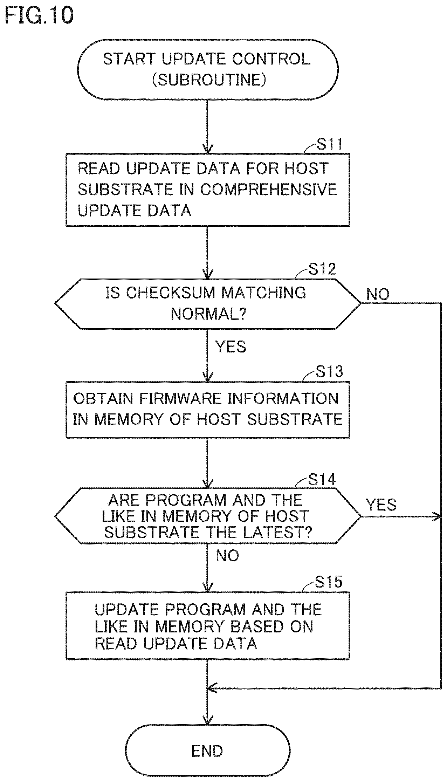

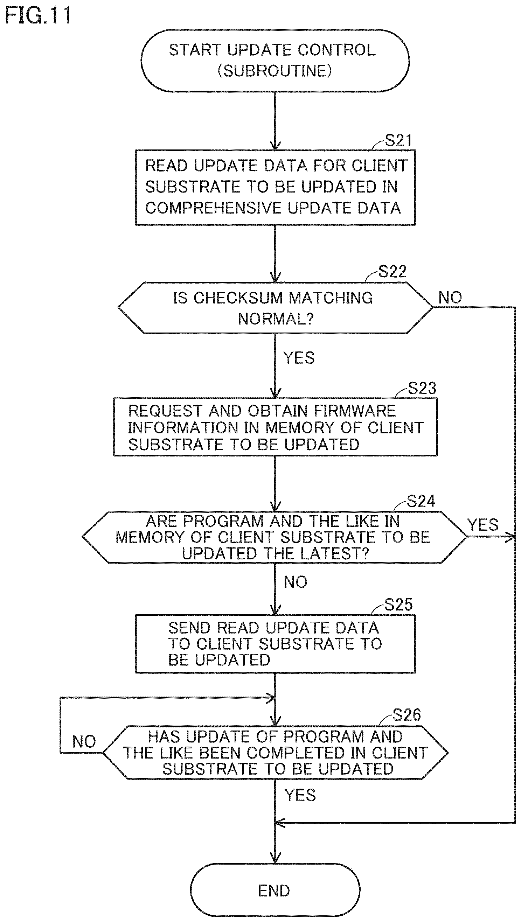

The host substrate calculates the total number N of constituent substrates based on the constituent member table in the memory (S4). The host substrate executes a subroutine (subroutine in Fig. 10) for executing the update processing for the host substrate itself and a subroutine (subroutine in Fig. 11) for causing the client substrate to execute the update processing (S5).

The host substrate calculates the total number n (= N-1) of constituent substrates not yet subjected to the update processing (S6). The host substrate determines whether or not there is no constituent substrate not yet subjected to the update processing, i.e., n=0 (S7). If there is a constituent substrate not yet subjected to the update processing (NO in S7), the process returns to S5 again and the host substrate executes the subroutine.

On the other hand, if there is no constituent substrate not yet subjected to the update processing (YES in S7), the host substrate restarts various devices (S8) and ends the routine.

Next, Fig. 10 is a flowchart for describing the subroutine of the update control executed by the host substrate. Fig. 10 shows the subroutine for executing the update processing for the host substrate itself.

As shown in Fig. 10, the host substrate first reads the update data for the host substrate in the comprehensive update data specified in S3 in Fig. 8 (S11). In order to determine the validity of the read update data, the host substrate determines whether or not checksum matching is normal (S12). If the checksum matching is not normal (NO in S12), the host substrate ends the routine.

On the other hand, if the checksum matching is normal (YES in S12), the host substrate obtains the firmware information in the memory of the host substrate (S 13). The host substrate determines, by update determining unit 136, whether or not the program and the like in the memory of the host substrate are the latest, i.e., whether or not execution of the update processing is necessary, based on the obtained firmware information and the update data read in S 11 (S 14). If the program and the like in the memory of the host substrate are the latest, i.e., execution of the update processing is not necessary (YES in S14), the host substrate ends the routine.

On the other hand, if the program and the like in the memory of the host substrate are not the latest, i.e., execution of the update processing is necessary (NO in S 14), the host substrate executes the update processing for updating the program and the like in the memory, based on the update data read in S11 (S15), and ends the routine.

Next, Fig. 11 is a flowchart for describing the subroutine of the update control executed by the host substrate. Fig. 11 is the subroutine for causing the client substrate to execute the update processing.

As shown in Fig. 11, the host substrate first reads the update data for the client substrate in the comprehensive update data specified in S3 in Fig. 8 (S21). In order to determine the validity of the read update data, the host substrate determines whether or not checksum matching is normal (S22). If the checksum matching is not normal (NO in S22), the host substrate ends the routine.

On the other hand, if the checksum matching is normal (YES in S22), the host substrate requests and obtains the firmware information in the memory of the client substrate from the client substrate to be updated via the intra-apparatus network (S23). The host substrate determines, by update determining unit 136, whether or not the program and the like in the memory of the client substrate to be updated are the latest, i.e., whether or not execution of the update processing is necessary, based on the obtained firmware information and the update data read in S21 (S24). If the program and the like in the memory of the client substrate to be updated are the latest, i.e., execution of the update processing is not necessary (YES in S24), the host substrate ends the routine.

On the other hand, if the program and the like in the memory of the client substrate to be updated are not the latest, i.e., execution of the update processing is necessary (NO in S24), the host substrate sends the update data read in S21 to the client substrate to be updated specified in S2 in Fig. 8, by update data sending unit 134 (S25).

Thereafter, the host substrate determines whether or not the update processing of the program and the like has been completed in the client substrate to be updated (S26). The host substrate performs determination in S26 based on whether or not the information that can specify the completion of the update processing by updating unit 144 has been received from the client substrate via the intra-apparatus network. Thereafter, the host substrate ends the routine.

Medical care apparatus 10 of the present embodiment described above mainly includes the following configuration.

Medical care apparatus 10 of the present embodiment is a medical care apparatus in which a plurality of functions are selected and combined, the medical care apparatus including: at least one client substrate for performing a function; and a host substrate for causing each substrate to execute update processing for updating a program and the like that perform the function based on update data, the host substrate including: comprehensive update data obtaining unit 131 for obtaining comprehensive update data including pieces of update data of all substrates attachable to medical care apparatus 10; constituent substrate specifying unit 133 for specifying the client substrate attached to medical care apparatus 10; update data specifying unit 132 for specifying the update data corresponding to the client substrate specified by constituent substrate specifying unit 133 from the comprehensive update data obtained by comprehensive update data obtaining unit 131; update determining unit 136 for determining whether or not execution of the update processing is necessary, based on firmware information that can specify information about the program and the like of the client substrate specified by constituent substrate specifying unit 133 as well as the update data specified by update data specifying unit 132; and update data sending unit 134 for sending at least the update data specified by update data specifying unit 132 to the client substrate specified by constituent substrate specifying unit 133, when update determining unit 136 determines that execution of the update processing is necessary, the client substrate including: updating unit 144 for executing the update processing based on the update data sent from update data sending unit 134.

As a result, even when some trouble occurs in the program and the like of each constituent substrate and the update processing must be executed, individual preparation of the update data corresponding to each constituent substrate is unnecessary, and individual and sequential execution of the update processing on all of the constituent substrates is also unnecessary. Furthermore, the attached substrate is never specified incorrectly and the update data corresponding to the substrate is never selected incorrectly, and thus, various functions can be performed normally. As described above, the program and the like can be updated easily and correctly at a time even when the plurality of functions are selected and combined.

Of the substrates attached to medical care apparatus 10, the C substrate serves as the host substrate. Namely, as shown in Fig. 5, the units for executing the update control, such as comprehensive update data obtaining unit 131, constituent substrate specifying unit 133, update data specifying unit 132, update determining unit 136, and update data sending unit 134, are provided on the same substrate as that of function unit 139 for performing the function of the host substrate itself.

As a result, a common substrate can be used as the substrate for executing the update control and the substrate for performing the function. Therefore, space-saving substrate design can be achieved, as compared with the case of using different substrates as the substrate for executing the update control and the substrate for performing the function.

All pieces of update data corresponding to the sub-functions selectable in all substrates attachable to medical care apparatus 10 are stored in the comprehensive update data.

As a result, even when the substrate has a plurality of selectable sub-functions, and after the sub-function is selected, the substrate can perform the sub-function on condition that the update processing has been executed, like the D substrate serving as medical care instrument driving unit 14, the update processing can be executed easily and correctly by obtaining one piece of comprehensive update data.

All pieces of update data corresponding to the specifications of all substrates attachable to medical care apparatus 10 are stored in the comprehensive update data.

Furthermore, the serial information that can specify the specification of the constituent substrate is stored in the constituent member table in the memory of the host substrate, and the host substrate specifies the update data corresponding to the specification of the constituent substrate from the update data stored in the data section of the comprehensive update data, based on the serial information stored in the constituent member table and the serial information stored in the header section of the comprehensive update data.

As a result, even when the substrate has a plurality of specifications that perform the same function, the update data corresponding to the specification of the constituent substrate can be correctly specified from the comprehensive update data, and the update processing can be easily executed, by obtaining one piece of comprehensive update data.

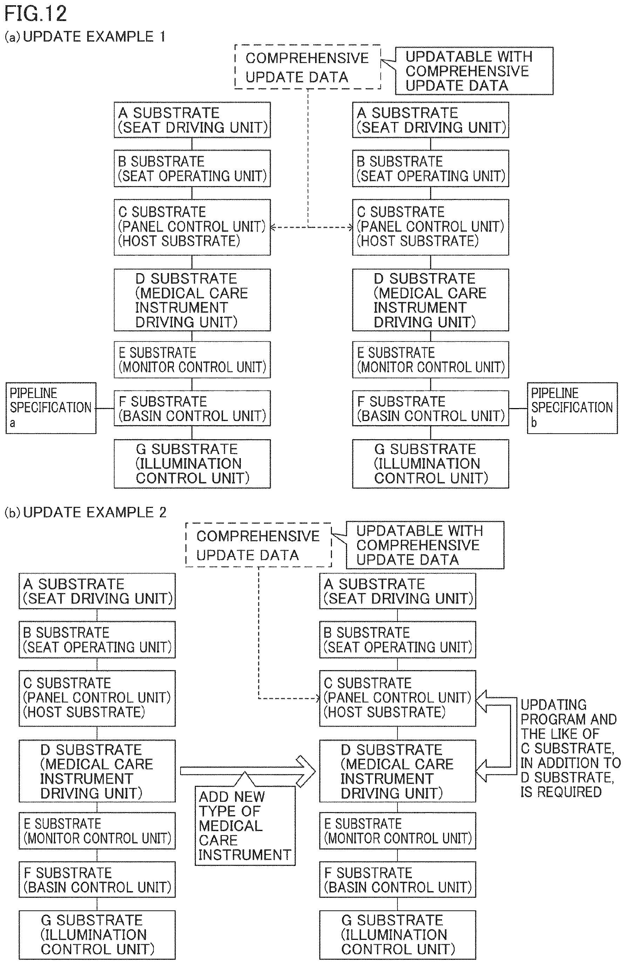

For example, Fig. 12 is a diagram for describing an update example of the program and the like. When a specification of a pipeline connected to the F substrate serving as basin control unit 16 is changed from a pipeline specification a to a pipeline specification b as in an update example 1 shown in Fig. 12(a), a program and the like corresponding to the pipeline specification a stored in a memory of the F substrate must be updated to a program and the like corresponding to the pipeline specification b subjected to the change. If the update data corresponding to each of the pipeline specification a and the pipeline specification b must be prepared individually or the update processing must be executed individually in accordance with the change of the pipeline specification, an abnormality such as, for example, water leakage may occur when the update data is selected incorrectly.

However, when the comprehensive update data storing all pieces of update data corresponding to the specifications of all substrates attachable to medical care apparatus 10 is used and the update data corresponding to the specification of the constituent substrate is specified based on the serial information as described above, the update processing can be executed easily and correctly.

In addition, at least one substrate performs a function related to another substrate, and can perform the function on condition that the update processing has been executed in the other substrate.

For example, when medical care instrument 4 is newly added as in an update example 2 shown in Fig. 12(b), added medical care instrument 4 cannot be driven unless a program and the like stored in a memory of the D substrate serving as medical care instrument driving unit 14 are updated. In addition, unless a program and the like stored in a memory of not only the D substrate but also the C substrate serving as panel control unit 13 are updated, a switch image for driving added medical care instrument 4 cannot be displayed and the operation for driving medical care instrument 4 cannot be accepted. Namely, when medical care instrument 4 is newly added, the update processing must be executed in both the D substrate and the C substrate. Furthermore, the update processing can be executed in the C substrate on condition that the D substrate has recognized added medical care instrument 4. Namely, the C substrate can perform the panel control function on condition that the update processing has been executed in the D substrate. If the update data corresponding to each of the C substrate and the D substrate must be prepared individually or the update processing must be executed individually on each of the C substrate and the D substrate, the function cannot be performed and the medical care itself may have to be interrupted when the update data is selected incorrectly, or when execution of the update processing on the C substrate is first tried although the update processing must be first executed on the D substrate.

However, in medical care apparatus 10 of the present embodiment, the update processing can be executed on all of the constituent substrates using one piece of comprehensive update data. Therefore, the program and the like can be updated easily and correctly.

In the checksum matching shown in S22 in Fig. 11, the host substrate determines the validity of the update data specified by update data specifying unit 132 before update data sending unit 134 sends the update data to the client substrate.

As a result, at the time of sending, an error of the update data can be detected, and it is possible to prevent the update data having the abnormality from being sent to the client substrate. Consequently, it is possible to prevent the update processing from being executed in the client substrate based on the update data having the abnormality.

### [Modification]

The present invention is not limited to the above-described embodiment, and various modifications and applications are further possible. A modification that is applicable to the present invention will be described below.

In the present embodiment, of the substrates attached to medical care apparatus 10, the C substrate serves as the host substrate. However, another substrate may serve as the host substrate. In addition, the present invention is not limited to medical care apparatus 10 in which one host substrate is provided. A plurality of host substrates may be provided. Furthermore, the substrates may be divided into groups based on a prescribed condition, and one host substrate may be provided in each group and the update control may be executed for each group. For example, of the A substrate to the W substrate, the C substrate and the P substrate may serve as the host substrates. Furthermore, the C substrate may execute the update control on the A substrate to the L substrate, and the P substrate may execute the update control on the M substrate to the W substrate.

In addition, a substrate (e.g., a dedicated substrate for executing the update control) different from the substrates for performing the functions attached to medical care apparatus 10 may serve as the host substrate. Namely, the substrate for executing the update control may be different from the substrates for performing the functions. With such a configuration, even when the substrate for executing the update control fails, removal of the substrates for performing the functions is unnecessary. Even when the substrates for performing the functions fail, removal of the substrate for executing the update control is unnecessary.

In the present embodiment, update data sending unit 134 of the host substrate sends the update data specified by update data specifying unit 132 to the client substrate specified by constituent substrate specifying unit 133. However, the present invention is not limited thereto. Update data sending unit 134 may send the comprehensive update data without any change to the client substrate specified by constituent substrate specifying unit 133. Namely, the host substrate may specify the client substrate attached to medical care apparatus 10, and send the comprehensive update data obtained from outside without any change to the specified client substrate. Then, the client substrate may specify, by itself, the update data for updating the program and the like stored in the memory, from the obtained comprehensive update data, and may execute the update processing based on the specified update data.

As shown in Fig. 5, in the present embodiment, only the host substrate includes update determining unit 136. However, the present invention is not limited thereto. For example, only the client substrate may include the update determining unit. In this case, the client substrate may receive the update data specified by update data specifying unit 132 or the comprehensive update data without any change from update data sending unit 134 of the host substrate, and may determine whether or not execution of the update processing is necessary, based on the received update data or the comprehensive update data. Namely, medical care apparatus 10 is a medical care apparatus in which a plurality of functions can be selected and combined, the medical care apparatus including: at least one client substrate for performing a function; and a host substrate for causing each substrate to execute update processing for updating a program and the like that perform the function based on update data, the host substrate including: comprehensive update data obtaining unit 131 for obtaining comprehensive update data including pieces of update data of all substrates attachable to medical care apparatus 10; constituent substrate specifying unit 133 for specifying the client substrate attached to medical care apparatus 10; update data specifying unit 132 for specifying the update data corresponding to the client substrate specified by constituent substrate specifying unit 133 from the comprehensive update data obtained by comprehensive update data obtaining unit 131; and update data sending unit 134 for sending at least the update data specified by update data specifying unit 132 to the client substrate specified by constituent substrate specifying unit 133, the client substrate including: the update determining unit for determining whether or not execution of the update processing is necessary, based on firmware information that is stored in firmware information storing unit 142 and can specify information about the program and the like as well as the update data sent from update data sending unit 134 of the host substrate; and updating unit 144 for executing the update processing based on the update data sent from update data sending unit 134, when the update determining unit determines that execution of the update processing is necessary.

In such medical care apparatus 10 as well, even when some trouble occurs in the program and the like of each constituent substrate and the update processing must be executed, individual preparation of the update data corresponding to each constituent substrate is unnecessary, and individual and sequential execution of the update processing on all of the constituent substrates is also unnecessary. Furthermore, the attached substrate is never specified incorrectly and the update data corresponding to the substrate is never selected incorrectly, and thus, various functions can be performed normally. As described above, the program and the like can be updated easily and correctly at a time even when the plurality of functions are selected and combined.

Both the host substrate and the client substrate may include the update determining unit. Regardless of whether only the client substrate includes the update determining unit or both the host substrate and the client substrate include the update determining units, the configuration of medical care apparatus 10 described with reference to Figs. 1 to 12 is applicable.

In the present embodiment, the host substrate executes the update control for updating the program and the like stored in the memory of the constituent substrate to the latest program and the like. However, the present invention is not limited thereto. For example, the program and the like stored in the memory of the client substrate may be forcibly rewritten into an old version of program and the like (e.g., an immediately preceding version of program and the like) based on the operator's external operation. In this case, the update data for updating the program and the like to the latest program and the like, and the update data for rewriting the program and the like into the previous version of program and the like may be included in the comprehensive update data. Furthermore, the processing in S 14 in Fig. 10 and the processing in S24 in Fig. 11 may be omitted.

In the present embodiment, the host substrate specifies the update data corresponding to the specification of the constituent substrate from the update data stored in the data section of the comprehensive update data, based on the serial information stored in the constituent member table and the serial information stored in the header section of the comprehensive update data. However, the present invention is not limited thereto. For example, in addition to the signature of the constituent substrate attached to medical care apparatus 10, the information that can specify the specification of the constituent substrate may be stored in the constituent member table.

In the present embodiment, by firmware information obtaining unit 135, the host substrate obtains the firmware information stored in firmware information storing unit 142 of the client substrate. However, the present invention is not limited thereto. For example, the firmware information of each constituent substrate attached to medical care apparatus 10 may be prestored in firmware information storing unit 137 of the host substrate, and firmware information obtaining unit 135 may obtain the firmware information of each constituent substrate from firmware information storing unit 137.

In the present embodiment, the medical care apparatus placed in a hospital and the like has been described by way of example. However, the present invention is not limited to such equipped medical care apparatus, and the present invention is also applicable to a portable medical care apparatus that can be carried. Namely, the present invention is applicable to any medical care apparatuses as long as they are medical care apparatuses in which a plurality of functions can be selected and combined.

It should be understood that the embodiment disclosed herein is illustrative and non-restrictive in every respect. The scope of the present invention is defined by the terms of the claims, rather than the description above, and is intended to include any modifications within the meaning and scope equivalent to the terms of the claims.

### REFERENCE SIGNS LIST

1 medical care chair; 2 basin unit; 3 tray table; 4 medical care instrument; 5 foot controller; 9 control device; 10 medical care apparatus; 11 seat driving unit; 12 operation unit; 13 panel control unit; 14 medical care instrument driving unit; 15 monitor control unit; 16 basin control unit; 17 illumination control unit; 131 comprehensive update data obtaining unit; 132 update data specifying unit; 133 constituent substrate specifying unit; 134 update data sending unit; 136 update determining unit; 142 firmware information storing unit; 144 updating unit; 145 function unit.

## Claims

1. A medical care apparatus (10) in which a plurality of functions are selected and combined, the medical care apparatus comprising:
at least one function unit (11, 12, 13, 14, 15, 16, 17) configured to perform a function; and
an update control unit (13) configured to control the at least one function unit to execute update processing for updating at least one of a program and a piece of data that perform the function based on prescribed update information,
the update control unit including:
an obtaining unit (131) configured to obtain comprehensive update information including the update information of the at least one function unit attachable to the medical care apparatus;
a function specifying unit (133) configured to specify the at least one function unit attached to the medical care apparatus;
an update information specifying unit (132) configured to specify the update information corresponding to the at least one function unit specified by the function specifying unit from the comprehensive update information obtained by the obtaining unit; and
a sending unit (134) configured to send at least the update information specified by the update information specifying unit to the at least one function unit specified by the function specifying unit,
the function unit including an updating unit (144) configured to execute the update processing based on the update information sent from the sending unit,
the update control unit or the at least one function unit further including an update determining unit (136) configured to determine whether or not execution of the update processing is necessary, based on the at least one of the program and the piece of data as well as the update information specified by the update information specifying unit.

2. The medical care apparatus according to claim 1, wherein
the update control unit (13) is provided on a substrate same as a substrate of the at least one function unit (13) attached to the medical care apparatus.

3. The medical care apparatus according to claim 1, wherein
the update control unit (13) is provided on a substrate different from a substrate of the at least one function unit (11, 12, 14, 15, 16, 17) attached to the medical care apparatus.

4. The medical care apparatus according to any one of claims 1 to 3, wherein
the at least one function unit (14) has a plurality of selectable sub-functions,
after the sub-function is selected, the at least one function unit (14) performs the sub-function on condition that the update processing has been executed based on the update information corresponding to the selected sub-function,
the comprehensive update information includes the update information corresponding to the selectable sub-functions, and
the update information specifying unit specifies the update information corresponding to the at least one function unit specified by the function specifying unit and the selected sub-function from the comprehensive update information obtained by the obtaining unit.

5. The medical care apparatus according to any one of claims 1 to 4, wherein
the at least one function unit (13, 16) has a plurality of specifications that perform the same function,
the function specifying unit further specifies the specification of the at least one function unit attached to the medical care apparatus, and
the update information specifying unit specifies the update information corresponding to the at least one function unit specified by the function specifying unit and the specification from the comprehensive update information obtained by the obtaining unit.

6. The medical care apparatus according to claim 5, further comprising
a storing unit (130) configured to store specification information that can specify the specification of the at least one function unit, wherein
the update information specifying unit specifies the update information corresponding to the at least one function unit specified by the function specifying unit and the specification from the comprehensive update information obtained by the obtaining unit, based on the specification information stored in the storing unit.

7. The medical care apparatus according to any one of claims 1 to 6, wherein
the at least one function unit includes a basic function unit (11, 12, 13, 14) configured to perform a basic function of the medical care apparatus, and an extended function unit (15, 16, 17) configured to perform an extended function of the medical care apparatus.

8. The medical care apparatus according to any one of claims 1 to 7, wherein
the at least one function unit includes a first function unit (14) configured to perform a prescribed function, and a second function unit (13) configured to perform a function related to the first function unit, and
the second function unit performs the function on condition that the update processing has been executed in the first function unit.

9. The medical care apparatus according to any one of claims 1 to 8, wherein
the update control unit further includes a validity determining unit (134) configured to determine validity of the update information specified by the update information specifying unit, before the sending unit sends the update information.

10. The medical care apparatus according to any one of claims 1 to 9, wherein
the update control unit obtains the comprehensive update information by the obtaining unit via a transmission path connected wiredly or wirelessly.

11. The medical care apparatus according to any one of claims 1 to 10, wherein
the obtaining unit is communicable with an other medical care apparatus and obtains, via communication, the comprehensive update information obtained by the obtaining unit of the other medical care apparatus.
